# EUROPEAN PATENT SPECIFICATION

(11) **EP 2 358 794 B1**
(45) Date of publication and mention of the grant of the patent: **03.07.2013**
(21) Application number: 09764243.3
(22) Date of filing: 01.12.2009
(51) Int. Cl.: C08J 5/12, C08K 3/00, C09J 11/04, C09J 133/08, A41G 3/00, A61L 24/00, A61L 27/18, B32B 7/12, B32B 27/08, B32B 27/40, B32B 37/00, B32B 3/26

(54) **METHOD FOR THE MANUFACTURE OF A MULTI-LAYER SUBSTRATE WHICH CAN BE BONDED TO THE SKIN TO FORM SELF-ADHESIVE HAIR PROSTHESES AND MULTI-LAYER PROSTHESES SO OBTAINED**
VERFAHREN ZUR HERSTELLUNG EINES MEHRSCHICHTIGEN SUBSTRATS, DAS ZUR BILDUNG VON SELBSTKLEBENDEN HAARPROTHESEN MIT DER HAUT VERBUNDEN WERDEN KANN, UND SO ERHALTENE MEHRSCHICHTIGE PROTHESEN
PROCÉDÉ DE FABRICATION D UN SUBSTRAT MULTICOUCHE QUI PEUT ÊTRE RELIÉ À LA PEAU POUR FORMER DES PROTHÈSES DE CHEVEUX AUTO-ADHÉSIVES ET PROTHÈSES MULTICOUCHES AINSI OBTENUES

(30) Priority: 02.12.2008 IT GE20080100
(43) Date of publication of application: 24.08.2011
(73) Proprietor: Sicurmatica Di Massimiliano Delli, Province of Firenze (IT)
(72) Inventor: DELLI, Massimiliano, I-50041 Calenzano Province of Firenze (IT)
(74) Representative: Porsia, Attilio
(86) International application number: PCT/EP2009/066118
(87) International publication number: WO 2010/063699

(56) References cited:
- EP-A- 1 693 191
- EP-A- 1 810 582
- EP-B- 1 427 453
- DE-B3- 10 316 156

## Description

This invention relates to a method for the manufacture of adhesive multi-layer substrates which can be bonded to the skin to form self-adhesive hair prostheses, and multi-layer prostheses so obtained.

At the present time various types of hair prostheses which are normally used by many tens of thousands of people affected by baldness are available on the market. These prostheses can be summarily allocated to two main categories: rigid and elastic.

Rigid prostheses are characterised by a substrate of composite material (polyurethane and/or silicone) into which the hairs are inserted; these are appreciably thick and rigid, being of the order of a few millimetres. These prostheses are generally custom-made on an expanded polyurethane cast obtained by filling an impression of the user's head and matching the shape of his baldness.

Elastic prostheses are made using an extremely soft and elastic polyurethane (from 20 to 300 microns) which are suitably preformed into a cap of standard size into which the hairs are inserted. The roots of the hairs projecting from the underside are secured to the substrate by a spread layer of polyurethane which embeds them. These "standard" prostheses are suitably cut to the shape of the user's typical baldness using a cast custom-made on customer's heads with a thin film of polyethylene and an adhesive layer with a glass fibre core. The elasticity of the substrate means that the shaped prostheses can be perfectly adjusted to the surface of the head.

In both cases, for both rigid and elastic prostheses, adhesion to the user's head is achieved through an adhesive bonding system. These systems may use adhesive tape or liquid adhesive spread with a brush.

The main disadvantage of these conventional prostheses/ head adhesion systems lies in the fact that in contact with sebaceous fat and the acidity of sweat the adhesive used tends to come loose, with the result that the prosthesis is progressively detached from the user's head.

For these reasons, and in addition the hygiene problems due to sebum and sweat remaining between the prosthesis and the skin, the prostheses have to be removed periodically for cleaning and replacement of the adhesive.

This "maintenance" operation, also known as the "hygienic phase", performs the function of rendering the skin and the prosthesis hygienic.

The problems deriving from the present technological situation for hair prostheses can be summarised in the main as: adhesion time, lack of an effective transpiration system, large thicknesses and long times for the hygienic phase.

As far as adhesion times are concerned, the systems presently in use allow periods between hygienic phases which vary from a few days up to 2/3 weeks, depending upon the pH of the sweat and the amount which the user sweats. In addition to this, conventional adhesives are attacked by physiological components of the user's skin such as sweat and sebum and ultimately detach from the head and bind to the hair, giving rise to an adverse aesthetic image and wholly losing adhesion.

As far as the problem of transpiration is concerned, it is pointed out that both adhesive strips and adhesive liquids form an impermeable film on the skin which wholly prevents transpiration, holding back sebum and sweat, thus encouraging significant bacterial growth, which in addition to being unhygienic produces unpleasant smells.

As far as prostheses are concerned, it is impossible to achieve effective transpiration and sufficient drainage of sebum and sweat using present manufacturing technology. Rigid prostheses, although drilled to permit the circulation of air between the prosthesis and the hair-bearing skin, are absolutely ineffective in ensuring any transpiration. Elastic prostheses are wholly impermeable to sweat, which remains between the prosthesis and the skin, because of the polyurethane spread to fix the hairs to the substrate and the subsequent application of liquid adhesives to provide adhesion.

In addition to this, these prostheses are of substantial thickness, some 400/600 microns, while the market requires continuous reductions in thickness to achieve greater more natural hair and greater comfort for users.

Finally, present-day technology provides that the time needed for the periodical hygienic phases is approximately 50/60 minutes, in addition to the time needed to cut the hair, that is an excessively long time.

From EP-A-1-1810582 a process for the production of an adhesive for a wig, made of urethane gel, is known. However, by the use of such an adhesive the above mentioned problem are not solved, and in particular the use of such an adhesive for bonding to the human skin hair prostheses will not allow the skin to transpire and will not contrast the bacterial growth.

The main object of this invention is therefore to provide a substrate which can be bonded to the skin to form self-adhesive hair prostheses suitable for producing a multi-layer prosthesis capable of overcoming the abovementioned disadvantages of the prostheses currently in use.

In particular, according to a principal characteristic of this invention, it provides a method for the manufacture of a substrate which can be bonded to the skin in order to form self-adhesive hair prostheses through the use of a liquid adhesive, comprising the stages of:
- adding up to 5% by weight of micronized silver powder or a stoichiometrically quantitative equivalent of Ag⁺ ions in relation to the total weight of the adhesive"
- intimately mixing the Ag powder and/or the Ag⁺ ions and the adhesive,
- blowing microbubbles of air into this liquid mixture during the mixing stage,
- spreading the said mixture on a thermoformable silicone film which can be quite easily or readily detached with the formation of a film having thicknesses of 5 to 80 microns in a single spreading operation.

According to a further feature of the invention it provides for application of a polyurethane membrane which allows transpiration through microperforation of the same to that film, and subsequently inserting hair onto that polyurethane membrane to form the hair prosthesis, and subsequent application of the said prosthesis onto one of the previously-shaped bondable substrates.

Further features and advantages of this invention will be more apparent in the course of the following description, made with reference to the appended drawings, provided by way of a non-restrictive example of this invention, in which:
Figure 1 diagrammatically illustrates in longitudinal cross-section a standard flat thermoformable adhesive substrate according to a first embodiment of this invention,
Figure 2 diagrammatically illustrates the substrate in Figure 1 after thermoforming, in axial longitudinal cross-section,
Figure 3 diagrammatically illustrates the application of a microperforated hair membrane to a thermoformable substrate of the type in Figure 1,
Figure 4 diagrammatically illustrates the substrate in Figure 3 after thermoforming,
Figure 5 diagrammatically illustrates application of the substrate in Figure 4 to a silicone head,
Figure 6 diagrammatically illustrates the insertion of hair onto the membrane for hair mounted on the silicone head,
Figure 7 diagrammatically illustrates permanent attachment of the hair to the membrane for hair in Figure 6, with microperforation of the attachment layer,
Figure 8 diagrammatically illustrates coupling of the membrane for hair in Figure 6 with the substrate in Figure 2,
Figures 9 to 12 diagrammatically illustrate four variants for attachment of the finished product.

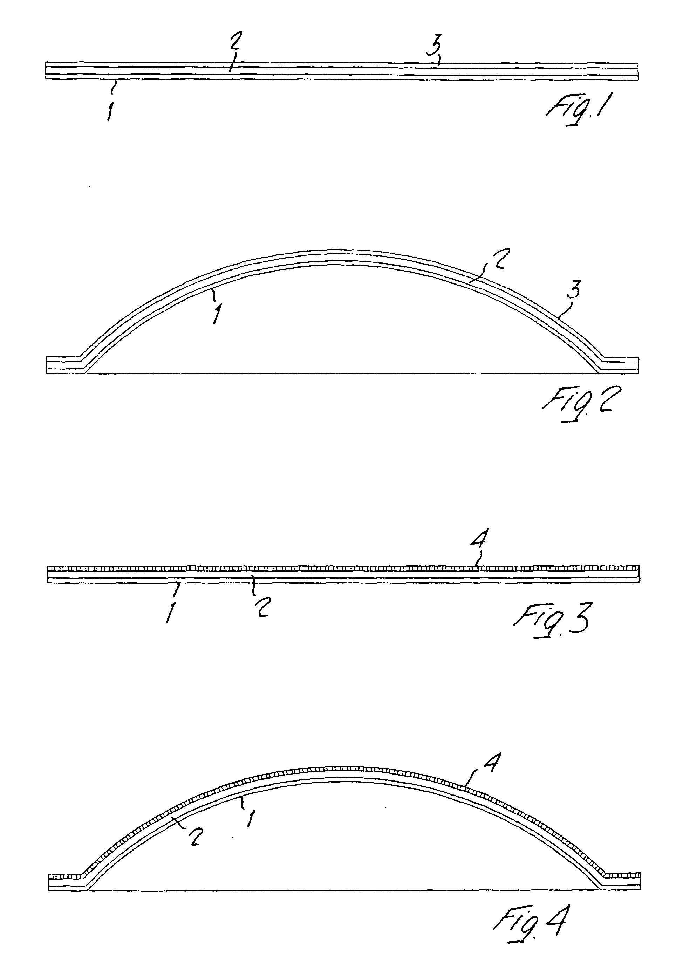

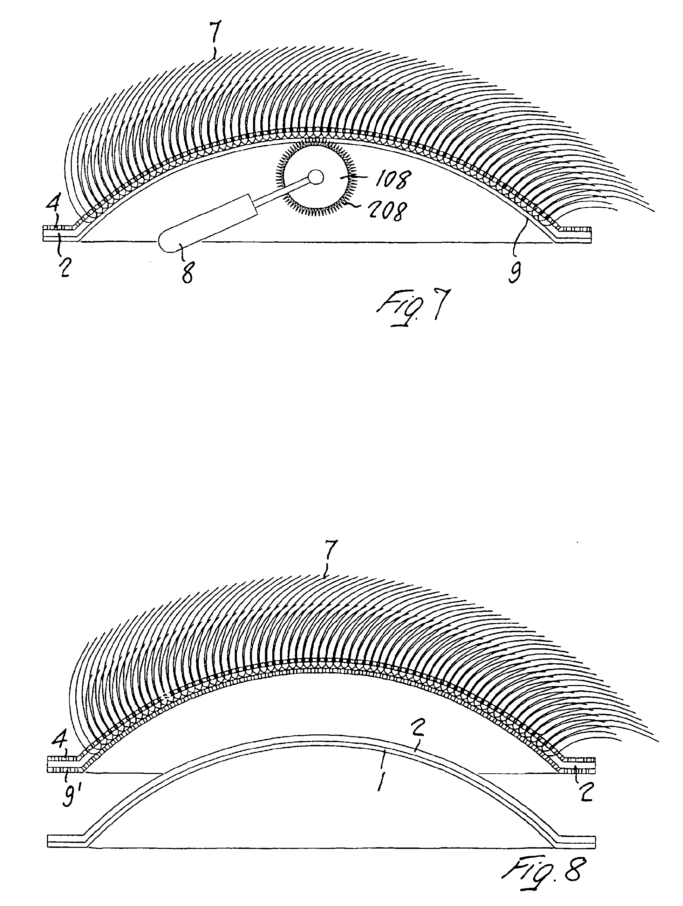

With reference to Figure 1 in the drawings, 1 indicates a silicone polyethylene film which is easily detachable and thermoformable at a temperature of approximately 60°-80°C, that is a temperature which can be obtained even using an ordinary professional phon drier. A hypoallergenic acrylic liquid adhesive, such as for example an ethyl acetate in silicone base, such as that for example known commercially as "Ultra Hold Adhesive" produced by Walker Tape Jordan, Utah, U.S.A. or a water-based polyvinyl acetate such as for example that known commercially as "Aqua Bond" or "Perfector Plus" produced by Hair & Compounds, California, U.S.A., is spread onto the said film. However, because these adhesives are wholly impermeable and prevent transpiration of any kind, encouraging bacterial growth caused by sweat and sebum, to overcome this problem it is provided according to the invention that air be blown through a microperforated tube into the liquid adhesive during the stage in which it is mixed, with the formation of microbubbles containing a mixture of air and residues of solvent vapour within them trapped within the adhesive. In addition to this, again with a view to preventing the process of bacterial growth, which apart from rendering the effect of sebum and sweat more aggressive to the adhesive, is the cause of the bad smell which is typical of prostheses used for long periods of time, an antibacterial product having a bacteriostatic action is incorporated into the adhesive. For this purpose provision is made for incorporating micronized silver powder in quantities up to 5% by weight in relation to the total weight of the adhesive, or a stoichiometrically equivalent quantity of silver ions, such as for example those provided by the product known commercially by the name of "lonpure" produced by lshizuka Glass Co. Ltd., Japan, is incorporated into the adhesive in the mixing stage. The liquid adhesive so obtained is spread in thicknesses varying from 20 microns to 80 microns in a single layer and from 100 microns to 400 microns in successive layers. The film so obtained is caused to pass through a stove which by heating to a temperature of 70°-80° encourages evaporation of the excess solvent from the adhesive creating an extremely dry adhesive. The layer of adhesive 2 so obtained is covered by a readily-detachable silicone film 3 allowing transpiration and is passed through a system of rollers which renders the spread material uniform, yielding a strongly adhesive membrane which is strongly resistant to aggression by sweat and sebum. To render the substrate for the prosthesis capable of transpiration, suitable equipment (see device 8 in Figure 7) comprising a small roller 108 on which hundreds of tapering needles 208 with diamond points having a diameter of 1 micron has been produced. This roller is passed over the membrane and thus creates thousands of micropores of tapering shape which because of the elasticity of the material are normally closed and do not allow water to pass through. When the pressure due to sweat increases, as a result of their special tapering shape these microholes act like real pores and open, allowing excess sweat and sebum to escape.

At this point, to form the prosthesis the flat sheet formed in this way is cut to a suitable size for "standard" prostheses, which are suitable for almost all types of baldness; these cut portions are heated to temperatures of the order of 80°C, and using a press in the form of male and female spherical cups are curved so as to obtain a cap shape, as illustrated in Figure 2.

At this point, as more particularly illustrated in Figures 3 and 4, a new sandwich of the type of that in Figure 1 in which film 3 is replaced by a thin polyurethane membrane 4 (approximately 25 microns thick) which has been rendered capable of transpiration through microperforation as previously described is prepared. This new sandwich is again heated to approximately 80°C and placed in a heated male/female press with a cup reproducing the shape of the head, to impart a cap shape to it, as illustrated in Figure 4.

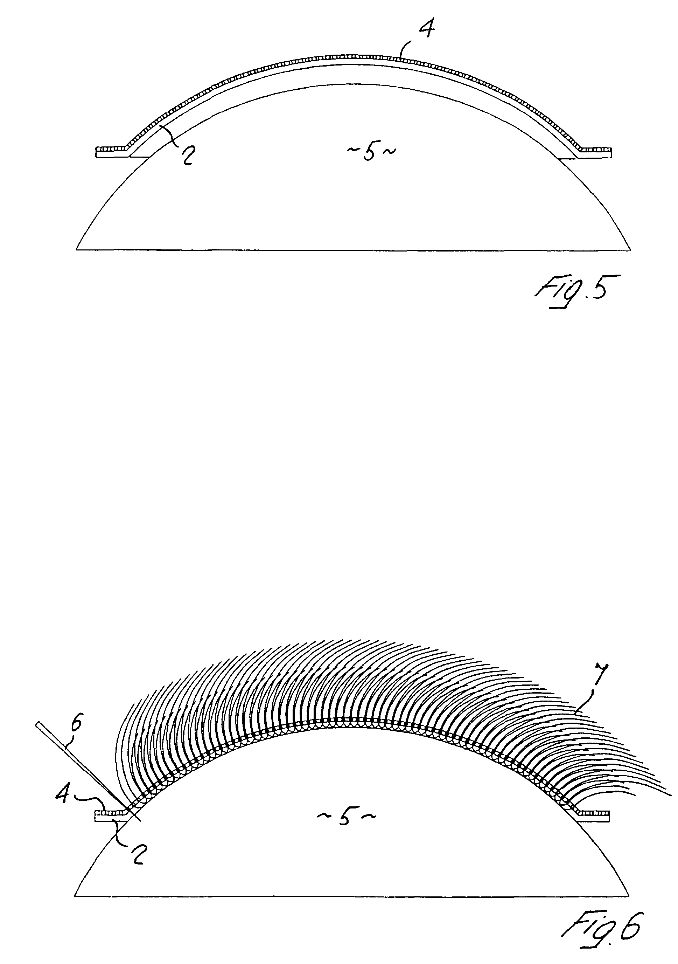

At this point protective film 1 is removed from the sandwich so obtained, and is positioned on a non-stick silicone head 5, as illustrated in Figure 5, after which hairs 7 are inserted into polyurethane film 4, as illustrated in Figure 6, using a needle 6. After this operation the prosthesis is easily removed from head 5 and a layer of liquid polyurethane is spread over the roots of the hairs to secure them. As it solidifies the polyurethane forms a film 9 which is suitably microperforated using device 8, as illustrated in Figure 7, to restore transpiration.

At this point the product in Figure 7 can be attached to the product in Figure 2, from which protective liner 3 has been removed, to obtain the finished product, as shown in Figure 8, ready for application to the user's head.

When the prosthesis is applied to the skin, a tractive force is exerted upon it, breaking the bubbles, creating openings in the adhesive, which when the prosthesis is applied to the user's head allows the skin the transpire.

The surface bubbles which remain partly closed create hemispherical cups which when applied to the skin are subjected to a pressure which allows the air present within them to escape, creating a vacuum effect like small suction cups.

In addition to this, the bubbles which remain enclosed within the adhesive, still full of air/solvent mixture, open up when attacked by sebum and/or sweat and carry out an oxidation reaction on the molecules penetrating within them, appreciably reducing their acidity and thus their ability to attack the adhesive.

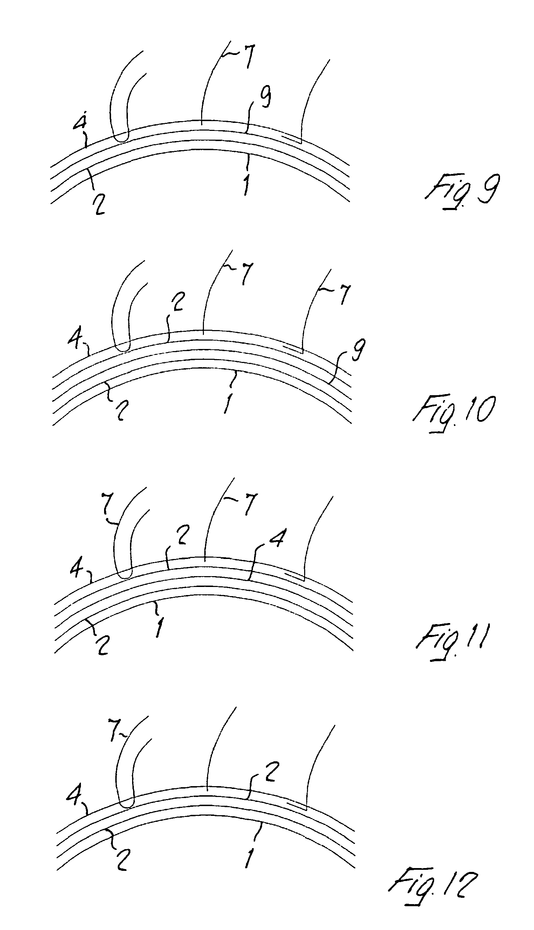

Figures 9 to 12 illustrate diagrammatically four possible methods of attachment between the product in Figure 7 and that in Figure 2. Figure 9 shows the attachment illustrated in greater detail in Figure 8.

In the variant in Figure 10, a layer of liquid polyurethane 9 is inserted between the product in Figure 7 and that in Figure 2. In a variant in Figure 11 a polyurethane membrane 4 is inserted between the product in Figure 7 and that in Figure 2, and finally, in the variant in Figure 12 liquid polyurethane film 9 in Figure 9 is replaced by an adhesive membrane 2.

Of course this invention is not restricted to the embodiments illustrated and described, which are given purely by way of examples of the invention. Thus for example the product in Figure 1 can instead be prepared using one or more uniform spread layers through the application of adhesive 2 to silicone film 1 in thin parallel threads separated by empty spaces, overlapping the sheets so prepared rotating them by 90° with respect to those beneath, obtaining a sandwich characterised by a weave of the fabric type comprising full parts and empty parts in which the full parts provide adhesion and the empty parts permit transpiration.

## Claims

1. Method for the manufacture of a substrate which can be bonded to the skin in order to form self-adhesive hair prostheses through the use of a liquid adhesive, comprising the stages of:
- adding up to 5% by weight of micronized silver powder or a stoichiometrically quantitative equivalent of Ag⁺ ions in relation to the total weight of the adhesive"
- intimately mixing the Ag powder and/or the Ag⁺ ions and the adhesive,
- blowing microbubbles of air into that liquid mixture during the mixing stage,
- spreading the mixture (2) so obtained over a thermoformable quite easily or readily detached silicone film (1) in thicknesses of 5 to 80 microns in a single spread layer.

2. Method according to Claim 1, in which the said adhesive is an acrylic adhesive.

3. Method according to Claim 1 or 2, comprising the subsequent stages of:
- subsequently spreading the said mixture (2) of adhesive and Ag onto the said thermoformable silicone film (1) until thicknesses of between 100 and 400 microns are obtained,
- applying a fairly easily or readily detachable silicone film (3) to the film so obtained.

4. Method according to Claim 3, comprising a further stage of heating the sandwich so formed to temperatures of the order of 60-80°C in a male/female press reproducing the typical shape of the head, in such a way as to impart the shape of a cap.

5. Method according to Claims 1 or 2, comprising the further stages of:
- applying to that film a polyurethane membrane (4) which has been rendered capable of transpiration through microperforation,
- heating the sandwich so formed to temperatures of the order of 60-80°C in a male/female press reproducing the standard shape of the head, in such a way as to impart the shape of a cap,
- removing the thermoformable film (1) and positioning it on a silicone head (5),
- inserting hair (7) into the said polyurethane membrane (4) forming the hair prosthesis.

6. Method according to Claim 5, in which after removal of the said prosthesis from the said head (5) a layer of liquid polyurethane (9) is spread onto the side of the prosthesis where the hair roots are located.

7. Method according to Claim 2, in which the said acrylic adhesive is a liquid hypoallergenic ethyl acetate in a silicone base, or a liquid hypoallergenic polyvinyl acetate in a water base.

8. Multi-layer adhesive prosthesis for hair obtained by joining the prosthesis mentioned in Claims 5 and/or 6 to a substrate which can be bonded to the skin according to any one of Claims 1 to 4.

## Patentansprüche

1. Verfahren zur Herstellung eines Substrats, das zur Bildung von selbstklebenden Haarprothesen durch Verwendung eines flüssigen Klebstoffes mit der Haut verbunden werden kann, die jeweils folgenden Stufen umfasst:
- Die Hinzufügung von bis zu 5% in Gewicht an mikronisiertem Silberpulver beziehungsweise eines stöchiometrisch quantitatives Äquivalents von Ag⁺Ionen im Verhältnis zum Gesamtgewicht des Klebstoffs;
- Die innige Mischung des Ag Pulvers und/oder der Ag⁺Ionen und des Klebstoffs;
- Das Einblasen von Mikroluftblasen in diese flüssige Mischung während des Mischungsverfahrens;
- Das Auftragen der so erhaltenen Mischung (2) auf einen wärmeformbaren, einfach oder schnell ablösbaren Silikonfilm (1) in der Stärke von 5 bis 80 Mikron in einer einzigen Schicht.

2. Verfahren gemäß Anspruch 1, bei der der genannte Klebstoff ein Acryl klebstoff ist.

3. Verfahren gemäß Anspruch 1 oder 2, die folgende anschließende Stufen umfasst:
- Das anschließende Auftragen der genannten Mischung (2) aus Klebstoff und Ag auf den genannten wärmeformbaren Silikonfilm (1), bis eine Stärke zwischen 200 und 400 Mikron erreicht wird,
- Die Aufbringung eines leicht oder schnell entfernbaren Silikonfilms (3) auf den so erhaltenen Film.

4. Verfahren gemäß Anspruch 3, die eine weitere Stufe der Erhitzung des so gebildeten Schichtwerkstoffs auf eine Temperatur von 60-80° C in einer Positiv-/Negativpresse umfasst, welche die typische Form des Kopfs nachbildet, und zwar so, dass ihm die Form einer Haube verliehen wird.

5. Verfahren gemäß den Ansprüchen 1 oder 2, die folgende Stufen umfasst:
- Die Aufbringung einer Polyurethanmembrane (4) auf diesen Film, welche durch entsprechende Mikroperforation atmungsaktiv gemacht wurde,
- Die Erhitzung des so entstandenen Schichtwerkstoffs auf Temperaturen von 60-80°C in einer Positiv-/Negativpresse, die die Standardform des Kopfs nachbildet, und zwar so, dass ihm die Form einer Haube verliehen wird,
- Die Entfernung des wärmeformbaren Films (1) und die entsprechende Positionierung auf einem Silikonkopf (5),
- Den Einsatz von Haar (7) in die genannte Polyurethanmembrane (4), die die Haarprothese herstellt.

6. Verfahren gemäß Anspruch 5, bei der nach der Entfernung der genannten Prothese von dem genannten Kopf (5) eine Schicht von flüssigem Polyurethan (9) auf die Seite der Prothese aufgetragen wird, an der sich jeweils die Haarwurzeln befinden.

7. Verfahren gemäß Anspruch 2, bei der der genannte Acrylklebstoff ein flüssiges, hypoallergenes Äthylazetat auf Silikonbasis oder ein flüssiges hypoallergenes Polyvinylazetat auf Wasserbasis ist.

8. Eine mehrschichtige selbstklebende Haarprothese, die erhalten wird, indem man die in den Ansprüchen 5 und/oder 6 erwähnte Prothese mit einem Substrat kombiniert, das mit der Haut gemäß einem beliebigen der Ansprüche von 1 bis 4 verbunden werden kann.

## Revendications

1. Procédé de fabrication d'un substrat qui peut être relié à la peau pour former des prothèses de cheveux auto-adhésives au moyen de l'utilisation d'un adhésif liquide, comprenant les étapes :
- d'adjonction jusqu'à 5 % en poids de poudre d'argent micronisée ou d'une quantité équivalente d'un point de vue stoechiométrique d'Ag⁺ions par rapport au poids global de l'adhésif,
- de mélange d'un point de vue intime de la poudre d'Ag et/ou des Ag⁺ions et de l'adhésif
- de soufflage de microbulles d'air à l'intérieur de ce mélange liquide au cours de l'étape de mélange,
- de répartition du mélange (2) ainsi obtenu sur un film de silicone thermo-formable se détachant très facilement ou très rapidement (1) en épaisseurs variant entre 5 et 80 microns en une seule couche.

2. Procédé selon la revendication 1, **caractérisé en ce que** ledit adhésif est un adhésif acrylique.

3. Procédé selon la revendication 1 ou 2, **caractérisé en ce qu'**il comprend les étapes ultérieures :
- de répartition ultérieure dudit mélange (2) d'adhésif et d'Ag sur ledit film de silicone thermo-formable (1) jusqu'à ce que des épaisseurs comprises entre 100 et 400 microns soient obtenues,
- d'application d'un film de silicone se détachant assez facilement ou assez rapidement (3) sur le film ainsi obtenu.

4. Procédé selon la revendication 3, **caractérisé en ce qu'**il comprend une étape supplémentaire de chauffage du sandwich ainsi formé à des températures de l'ordre de 60-80°C dans une presse mâle-femelle reproduisant la forme typique de la tête, de manière à conférer la forme d'une calotte.

5. Procédé selon la revendication 1 ou 2, **caractérisé en ce qu'**il comprend les étapes ultérieures :
- d'application sur ce film d'une membrane polyuréthane (4) qui a été rendue apte à la transpiration par l'intermédiaire de la micro-perforation,
- de chauffage du sandwich ainsi formé à des températures de l'ordre de 60-80°C dans une presse mâle-femelle reproduisant la forme standard de la tête, de manière à conférer la forme d'une calotte,
- de retrait du film thermo-formable (1) et de placement de celui-ci sur une tête en silicone (5),
- d'insertion des cheveux (7) à l'intérieur de ladite membrane polyuréthane (4) formant la prothèse de cheveux.

6. Procédé selon la revendication 5, **caractérisé en ce qu'**une couche de polyuréthane liquide (9) est étendue sur le côté de la prothèse où les racines des cheveux sont placées, après le retrait de ladite prothèse de ladite tête (5).

7. Procédé selon la revendication 2, caractérisé en ce ledit adhésif acrylique est de l'acétate d'éthyle liquide hypoallergénique dans une base de silicone, ou de l'acétate de polyvinyle liquide hypoallergénique dans une base aqueuse.

8. Prothèse de cheveux multicouches adhésive obtenue en reliant la prothèse comme revendiqué dans la revendication 5 et/ou 6 à un substrat qui peut être relié à la peau selon une revendication quelconque parmi les revendications allant de 1 à 4.
